# EUROPÄISCHE PATENTSCHRIFT

(11) **EP 0 978 500 B1**
(45) Veröffentlichungstag und Bekanntmachung des Hinweises auf die Patenterteilung: **07.05.2003**
(21) Anmeldenummer: 99114920.4
(22) Anmeldetag: 30.07.1999
(51) Int. Cl.: C07C 51/083, C07C 59/88

(54) **Verfahren zur Herstellung von 4-(4'-Chlorbiphenyl-4-yl)-4-keto-2-methylenbuttersäure**
Process for the preparation of 4-(4'-chlorobiphenyl-4-yl)-4-keto-2-methylenbutyric acid
Procédé pour la préparation d'acide 4-(4'-chlorobiphenyl-4-yl)-4-keto-2-methylenbutyrique

(30) Priorität: 05.08.1998 DE 19835359
(43) Veröffentlichungstag der Anmeldung: 09.02.2000
(73) Patentinhaber: Clariant GmbH, 65929 Frankfurt am Main (DE)
(72) Erfinder: Meudt, Andreas, Dr., 65439 Flörsheim-Weilbach (DE); Pressler, Wilfried, Dr., 65779 Kelkheim (DE); Nörenberg, Antje, Dr., 64572 Büttelborn (DE); Haber, Steffen, Dr., 76829 Landau/Pfalz (DE); Erbes, Michael, 65931 Frankfurt (DE); Cosmo, Robert, Dr., 64291 Darmstadt (DE)

(56) Entgegenhaltungen:
- DE-A- 2 513 157
- US-A- 5 789 434
- H. COUSSE ET AL: "Synthèse, structure et activité hypocholestérolémiente d'une série d'acides gamma-aryl, gamma-oxo butyriques substitués et dérivés" EUR. J. MED. CHEM., Bd. 22, 1987, Seiten 45-57, XP000560283 Paris

## Beschreibung

Die Erfindung betrifft ein neues Verfahren zur Herstellung von 4-(4'-Chlorbiphenyl-4-yl)-4-keto-2-methylenbuttersäure der nachstehenden Formel (I).

4-(4'-Chlorbiphenyl-4-yl)-4-keto-2-methylenbuttersäure ist ein bedeutsames Zwischenprodukt für die Synthese von Matrix-Metalloendoproteinase-Inhibitoren (MMP), die als Wirkstoffe zur Verhinderung der Metastasierung maligner Tumoren eingesetzt werden. Die Verbindung neigt jedoch zur Isomerisierung zu konjugierten Isomeren. Diese Reaktion wird durch Basenspuren, starke Säuren sowie thermische Belastung induziert.

Aufgrund der Bedeutung von 4-(4'-Chlorbiphenyl-4-yl)-4-keto-2-methylenbuttersäure der Formel (I) existieren eine ganze Reihe von Vorschriften zur Herstellung dieser Verbindung. Alle bekannten Synthesen der Verbindung gehen dabei von 4-Chlorbiphenyl der Formel (II) aus, das mit AlCl₃ und Itaconsäureanhydrid der Formel (III) umgesetzt wird.

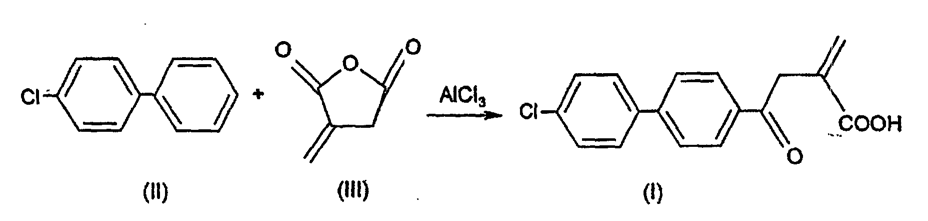

J.-P. Rieu et al. (J. Pharm. Sci. 1980, 69, 49) führen die Umsetzung im Lösungsmittel Dichlormethan durch. Nach wäßriger Aufarbeitung, Abdestillieren des Lösungsmittels und Umkristallisieren aus dem elffachen Volumen Ethylacetat erhält man das Produkt in einer Ausbeute von nur 52 %.

H. Cousse et al. (Eur. J. Med. Chem. 1987, 22, 45-57) verwenden als Solvens 1,2-Dichlorethan oder Dichlormethan. Nach wäßriger Aufarbeitung wird die organische Phase mit Ethanol und Aceton vermischt, woraufhin das Produkt ausfällt, welches abgesaugt und umkristallisiert wird. Die angegebenen Ausbeuten betragen allerdings nur 46 %.

Auch in der DE-A-25 13 157 werden Biphenyle und Halogenbiphenyle mit ltaconsäureanhydrid in Gegenwart eines Lewis-Säure-Katalysators nach Friedel-Crafts acyliert. Als Lösungsmittel findet hier das stark toxische 1,1,2,2-Tetrachlorethan Verwendung. Ausbeuten sind für die Acylierung von 4-Chlorbiphenyl nicht angegeben.

Der Erfindung lag die Aufgabe zugrunde, ein industriell durchführbares Verfahren zur Herstellung von 4-(4'-Chlorbiphenyl-4-yl)-4-keto-2-methylenbuttersäure bereitzustellen, das die genannten, hochtoxischen chlorierten aliphatischen Kohlenwasserstoffe vermeidet und das Zielprodukt in guten Ausbeuten liefert. Des weiteren sollte das zu entwickelnde Verfahren möglichst frei von technisch aufwendigen Reinigungsprozeduren wie Umkristallisation oder Chromatographie sein.

Überraschenderweise wurde gefunden, daß die Acylierung von 4-Chlorbiphenyl mit Itaconsäureanhydrid unter Katalyse mit AlCl₃ in aromatischen Lösungsmitteln zu 4-(4'-Chlorbiphenyl-4-yl)-4-keto-2-methylenbuttersäure in sehr guten Ausbeuten führt. Trotz der bereits erwähnten hohen Labilität wird das Zielprodukt überraschenderweise in sehr hoher Reinheit erhalten. Weiterhin überraschend ist, daß unter den erfindungsgemäßen Bedingungen keine Acylierung des Lösungsmittels beobachtet wird.

Gegenstand der vorliegenden Erfindung ist daher ein Verfahren zur Herstellung von 4-(4'-Chlorbiphenyl-4-yl)-4-keto-2-methylenbuttersäure der Formel (I) 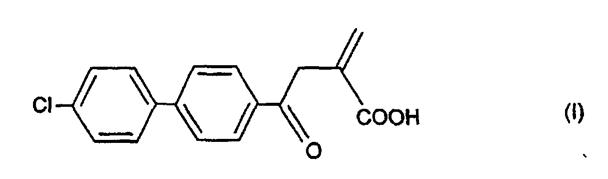 durch Friedel-Crafts-Acylierung von 4-Chlorbiphenyl mit Itaconsäureanhydrid, dadurch gekennzeichnet, daß die Friedel-Crafts-Acylierung in aromatischen Lösungsmitteln bei einer Temperatur von -20 bis +80°C durchgeführt wird.

Geeignete aromatische Lösungsmittel sind vorzugsweise solche, die unter den erfindungsgemäßen Bedingungen wesentlich langsamer acyliert werden als 4-Chlorbiphenyl, beispielsweise Benzonitril, Nitrobenzol, Brom- und Chloraromaten. Besonders bevorzugt sind o-Dichlorbenzol, Chlorbenzol und p-Chlortoluol. Auch Mischungen dieser Lösungsmittel können eingesetzt werden.

Die Ausgangsprodukte 4-Chlorbiphenyl und Itaconsäureanhydrid werden zweckmäßigerweise im molaren Verhältnis von 0,8:1 bis 1,2:1, vorzugsweise 0,9:1 bis 1,1:1, umgesetzt.

Als Friedel-Crafts-Katalysator wird beispielsweise AlCl₃ eingesetzt. Die Menge an Friedel-Crafts-Katalysator beträgt zweckmäßigerweise 1,0 bis 5 Mol je Mol itaconsäureanhydrid, vorzugsweise 2,01 bis 2,7 Mol je Mol ltaconsäureanhydrid.

In einer bevorzugten Ausführungsform werden 4-Chlorbiphenyl und Itaconsäureanhydrid im Lösungsmittel gelöst und diese Lösung zu einer Suspension des Friedel-Crafts-Katalysators im Lösungsmittel zudosiert, zweckmäßigerweise bei einer Temperatur von -20 bis +80°C, vorzugsweise bei -10 bis +55°C, besonders bevorzugt bei 0 bis 45°C.

Es ist auch möglich, eine feste Mischung der genannten Reaktanden in vorgelegtes Lösungsmittel einzudosieren. Ebenso ist es möglich, die Reaktanden in fester Form getrennt zuzudosieren. Die Dosierzeit oder Zutropfzeit kann zwischen 1 Minute und 12 Stunden, bevorzugt zwischen 0,5 und 4 Stunden liegen.

Die verwendete Lösungsmittelmenge (Summe aus Lösungsmittel in der Vorlage und dem zur Lösung der Reaktanden verwendeten Lösungsmittel) kann in weiten Grenzen variiert werden, ebenso das Verhältnis der Lösungsmittelmengen in der Vorlage zu der Lösungsmittelmenge, die für die Auflösung der Edukte dient. Zweckmäßig sind Gesamt-Lösungsmittelmengen zwischen 0,5 und 20 Gewichtsteilen je Gewichtsteil 4-Chlorbiphenyl, bevorzugt zwischen 8 und 16, besonders bevorzugt zwischen 11 und 15 Gewichtsteilen, je Gewichtsteil 4-Chlorbiphenyl.

Nach Beendigung des Zutropfens oder der Dosierung wird das Reaktionsgemisch 0,5 bis 24 Stunden, vorzugsweise 1 bis 6 Stunden, nachgerührt, vorzugsweise bei Temperaturen von 0 bis 55°C.

Anschließend wird mit Wasser, verdünnter wäßriger Salzsäure oder verdünnter wäßriger Schwefelsäure hydrolysiert.

Die Hydrolyse geschieht zweckmäßigerweise durch Eintragen der Reaktionsmischung in Wasser, Salzsäure oder Schwefelsäure, die vorzugsweise 0 bis 35 gew.-%ig sind, wobei Temperaturen zwischen 0 und 80°C bevorzugt sind. Die wäßrige Phase wird abgetrennt und die organische Phase ein- oder mehrfach mit verdünnter Salzsäure oder Schwefelsäure (0 bis 35 gew.-%ig) gewaschen. Das im Lösungsmittel suspendiert vorliegende Produkt wird abfiltriert und mit geeigneten Lösungsmitteln ein- oder mehrfach gewaschen und getrocknet. Geeignete Lösungsmittel sind solche, die sich gegenüber dem Reaktionsprodukt inert verhalten und eine Löslichkeit für das Produkt von unter 5 % aufweisen, beispielsweise o-Dichlorbenzol, Chlorbenzol, Ethanol oder Ethanol-Wasser-Mischungen.

Im allgemeinen werden nahezu quantitative Umsetzungen zum gewünschten Produkt erzielt.

Weiterhin ist es vorteilhaft, die Mutterlauge mit einer Lösung eines geeigneten Komplexbildners, beispielsweise einem mehrzähnigen Komplexbildner wie Ethylendiamintetraessigsäure zu waschen, wobei eventuell im Produkt enthaltenes Aluminium komplexiert und eine Isomerisierung des Produktes zu konjugierten Isomeren verhindert werden kann. Die Mutterlauge kann eingeengt werden, wodurch die Ausbeute weiter erhöht werden kann.

Die so erhaltene 4-(4'-Chlorbiphenyl-4-yl)-4-keto-2-methylenbuttersäure wird einer Trocknung unterzogen. Die Trocknung des Produktes kann bei Temperaturen zwischen 20°C und 110°C unter Drücken zwischen Normaldruck und 1 mbar durchgeführt werden.

Die folgenden Beispiele sollen die Erfindung erläutern, ohne sie zu begrenzen.

### Beispiel 1

212 g (1,59 mol) wasserfreies Aluminiumchlorid werden in 1200 g o-Dichlorbenzol vorgelegt und unter Rühren auf 45°C gebracht. Im Lauf einer Stunde tropft man eine 45°C warme Lösung von 78,5 g (0,7 mol) Itaconsäureanhydrid und 132 g (0,7 mol) 4-Chlorbiphenyl in 600 g o-Dichlorbenzol zu. Die Reaktionsmischung wird bei 45°C eine Stunde nachgerührt und anschließend in eine eiskalte Mischung aus 1000 g Wasser und 142 g Salzsäure (37 %) gegeben. Die wäßrige Phase wird abgetrennt. Man wäscht die organische Phase dreimal mit jeweils einer Mischung aus 1000 g Wasser und 50 g Salzsäure (37 %). Nach dreimaligem Waschen läßt man auf etwa 20°C abkühlen und saugt anschließend das farblose Produkt ab. Dieses wird anschließend zweimal mit jeweils 200 g o-Dichlorbenzol gewaschen. Das Produkt wird bei 40°C und ca. 100 mbar lösungsmittel- und wasserfrei getrocknet. Es werden 150 g (0,50 mol) 4-(4'-Chlorbiphenyl-4-yl)-4-keto-2-methylenbuttersäure (71 %) als farbloses, feinkristallines Pulver erhalten. Weiteres Produkt läßt sich durch Aufarbeitung der Mutterlauge gewinnen. Die Dichlorbenzolphase wird mehrfach mit 0,1 gew.-%iger wäßriger EDTA-Lösung gewaschen. Nach Abdestillieren des Dichlorbenzols im Vakuum wird durch Abkühlen des Rückstandes auf 0°C, Absaugen des farblosen Niederschlags und Umkristallisieren des Rückstandes weiteres Produkt erhalten und die Ausbeute auf > 85 % erhöht. (HPLC-Reinheit 99,3 - 99,6 %).

### Beispiel 2

212 g (1,59 mol) wasserfreies Aluminiumchlorid werden in 1800 g o-Dichlorbenzol vorgelegt und unter Rühren auf 45°C gebracht. Im Lauf einer Stunde wird eine feste Mischung aus 78,5 g (0,7 mol) Itaconsäureanhydrid und 132 g (0,7 mol) 4-Chlorbiphenyl zudosiert. Die Reaktionsmischung wird bei 45°C zwei Stunden nachgerührt und anschließend in eine eiskalte Mischung aus 900 g Wasser und 100 g Salzsäure (37 %) gegeben. Die wäßrige Phase wird abgetrennt. Man wäscht die organische Phase dreimal mit jeweils einer Mischung aus 1000 g Wasser und 25 g Salzsäure (37 %). Nach dreimaligem Waschen läßt man auf etwa 20°C abkühlen und saugt anschließend das farblose Produkt ab. Dieses wird anschließend zweimal mit jeweils 150 g Ethanol/Wasser 90:10 gewaschen. Das Produkt wird bei 80°C und ca. 110 mbar lösungsmittel- und wasserfrei getrocknet. Es werden 148 g (0,49 mol) 4-(4'-Chlorbiphenyl-4-yl)-4-keto-2-methylenbuttersäure (70 %) als farbloses, feinkristallines Pulver erhalten. Durch Aufarbeitung der Mutterlauge, wie in Beispiel 1 beschrieben, wird die Ausbeute auf 87 % erhöht. (HPLC-Reinheit 99,3 - 99,5 %).

### Beispiel 3

193,3 g (1,45 mol) wasserfreies Aluminiumchlorid werden in 1100 g o-Dichlorbenzol vorgelegt und unter Rühren auf 45°C gebracht. Im Lauf von drei Stunden tropft man eine 50°C warme Lösung von 80,9 g (0,72 mol) Itaconsäureanhydrid und 132 g (0,7 mol) 4-Chlorbiphenyl in 700 g o-Dichlorbenzol zu. Die Reaktionsmischung wird bei 25°C vier Stunden nachgerührt und anschließend in eine eiskalte Mischung aus 1000 g Wasser und 25 g Schwefelsäure (96 %) gegeben. Die wäßrige Phase wird abgetrennt. Man wäscht die organische Phase dreimal mit jeweils einer Mischung aus 1000 g Wasser und 15 g Schwefelsäure (96 %). Nach dreimaligem Waschen läßt man auf etwa 20°C abkühlen und saugt anschließend das farblose Produkt ab. Dieses wird anschließend dreimal mit jeweils 150 g Ethanol (96 %) gewaschen. Das Produkt wird bei 60°C und ca. 100 mbar lösungsmittel- und wasserfrei getrocknet. Es werden 148 g (0,49 mol) 4-(4'-Chlorbiphenyl-4-yl)-4-keto-2-methylenbuttersäure (70 %) als farbloses, feinkristallines Pulver erhalten. Durch Aufarbeitung der Mutterlauge, wie in Beispiel 1 beschrieben, wird die Ausbeute auf 83 % erhöht. (HPLC-Reinheit
99,6 - 99,8 %).

### Beispiel 4

21,2 g wasserfreies Aluminiumchlorid werden in 130 g Chlorbenzol vorgelegt und auf 0°C gekühlt. Im Lauf einer Stunde gibt man portionsweise eine feste Mischung aus 8,3 g ltaconsäureanhydrid und 13,2 g 4-Chlorbiphenyl zu. Die Reaktionsmischung wird bei 0°C drei Stunden nachgerührt und anschließend in Eiswasser gegeben. Das feste Reaktionsprodukt wird abfiltriert und zweimal mit Wasser (50 ml) gewaschen. Das Produkt wird bei Raumtemperatur und ca. 100 mbar lösungsmittel- und wasserfrei getrocknet. 4-(4'-Chlorbiphenyl-4-yl)-4-keto-2-methylenbuttersäure wird in einer Ausbeute von 75 % als farbloses, feinkristallines Pulver erhalten.

### Beispiel 5

21,2 g wasserfreies Aluminiumchlorid werden in 50 g Brombenzol vorgelegt und auf 0°C gekühlt. Im Lauf einer Stunde gibt man portionsweise eine feste Mischung aus 8,3 g Itaconsäureanhydrid und 13,2 g 4-Chlorbiphenyl zu. Die Reaktionsmischung wird bei 0°C zwei Stunden nachgerührt und anschließend in 100 g Eiswasser gegeben. Das feste Reaktionsprodukt wird abfiltriert und zweimal mit Wasser (50 ml) gewaschen. Nach Umkristallisation aus Ethylacetat wird das Produkt bei Raumtemperatur und ca. 100 mbar lösungsmittel- und wasserfrei getrocknet. 4-(4'-Chlorbipheny)-4-yl)-4-keto-2-methylenbuttersäure wird in einer Ausbeute von 50 % als farbloses, feinkristallines Pulver erhalten.

### Beispiel 6

21,2 g wasserfreies Aluminiumchlorid werden in 50 g p-Chlortoluol vorgelegt und unter Rühren auf 40°C erhitzt. Im Lauf einer Stunde gibt man portionsweise eine feste Mischung aus 8,3 g Itaconsäureanhydrid und 13,2 g 4-Chlorbiphenyl zu. Die Reaktionsmischung wird bei 40°C 30 Minuten nachgerührt und anschließend in 100 g Eiswasser gegeben. Das feste Reaktionsprodukt wird abfiltriert und zweimal mit Wasser (50 ml) gewaschen. Das Produkt wird bei Raumtemperatur und ca. 100 mbar lösungsmittel- und wasserfrei getrocknet. 4-(4'-Chlorbiphenyl-4-yl)-4-keto-2-methylenbuttersäure wird in einer Ausbeute von 70 % als farbloses, feinkristallines Pulver erhalten.

## Patentansprüche

1. Verfahren zur Herstellung von 4-(4'-Chlorbiphenyl-4-yl)-4-keto-2-methylenbuttersäure der Formel (I) 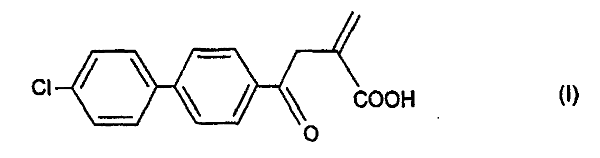 durch Friedel-Crafts-Acylierung von 4-Chlorbiphenyl mit Itaconsäureanhydrid, **dadurch gekennzeichnet, daß** die Friedel-Crafts-Acylierung in aromatischen Lösungsmitteln bei einer Temperatur von -20 bis +80°C durchgeführt wird.

2. Verfahren nach Anspruch 1, **dadurch gekennzeichnet, daß** das aromatische Lösungsmittel ein solches ist, das unter den Verfahrensbedingungen wesentlich langsamer acyliert wird als 4-Chlorbiphenyl.

3. Verfahren nach Anspruch 1 oder 2, **dadurch gekennzeichnet, daß** das aromatische Lösungsmittel ein Bromaromat, ein Chloraromat, Benzonitril oder Nitrobenzol ist.

4. Verfahren nach mindestens einem der Ansprüche 1 bis 3, **dadurch gekennzeichnet, daß** das aromatische Lösungsmittel o-Dichlorbenzol, Chlorbenzol oder p-Chlortoluol ist.

5. Verfahren nach mindestens einem der Ansprüche 1 bis 4, **dadurch gekennzeichnet, daß** AlCl₃ als Friedel-Crafts-Katalysator eingesetzt wird.

6. Verfahren nach mindestens einem der Ansprüche 1 bis 5, **dadurch gekennzeichnet, daß** nach der Friedel-Crafts-Acylierung eine Hydrolyse mit Wasser, wäßriger Salzsäure oder wäßriger Schwefelsäure durchgeführt wird.

7. Verfahren nach mindestens einem der Ansprüche 1 bis 6, **dadurch gekennzeichnet, daß** 4-Chlorbiphenyl und Itaconsäureanhydrid im aromatischen Lösungsmittel gelöst und diese Lösung zu einer Suspension des Friedel-Crafts-Katalysators im aromatischen Lösungsmittel zudosiert wird.

8. Verfahren nach mindestens einem der Ansprüche 1 bis 6, **dadurch gekennzeichnet, daß** 4-Chlorbiphenyl und Itaconsäureanhydrid in fester Form in eine Suspension des Friedel-Crafts-Katalysators im aromatischen Lösungsmittel zudosiert werden.

9. Verfahren nach Anspruch 7 oder 8, **dadurch gekennzeichnet, daß** die Dosierzeit zwischen 1 Minute und 12 Stunden, vorzugsweise zwischen 0,5 und 4 Stunden, liegt.

10. Verfahren nach Anspruch 9, **dadurch gekennzeichnet, daß** nach Beendigung der Dosierung 0,5 bis 24 Stunden, vorzugsweise 1 bis 6 Stunden, bei einer Temperatur zwischen 0 und 55°C nachgerührt wird.

11. Verfahren nach mindestens einem der Ansprüche 1 bis 10, **dadurch gekennzeichnet, daß** das nach der Hydrolyse im Lösungsmittel suspendiert vorliegende Produkt abfiltriert wird.

12. Verfahren nach Anspruch 11, **dadurch gekennzeichnet, daß** die nach der Filtration zurückbleibende Mutterlauge mit einer Lösung eines mehrzähnigen Komplexbildners gewaschen wird.

13. Verfahren nach Anspruch 12, **dadurch gekennzeichnet, daß** die mit einer Lösung eines mehrzähnigen Komplexbildners gewaschene Mutterlauge eingeengt und das im Lösungsmittel suspendiert vorliegende Produkt abfiltriert wird.

## Claims

1. A process for the preparation of 4-(4'-chlorobiphenyl-4-yl)-4-keto-2-methylenebutyric acid of the formula (I) 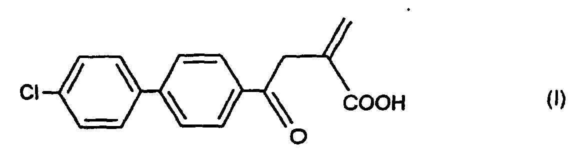 by Friedel-Crafts acylation of 4-chlorobiphenyl with itaconic anhydride, which comprises carrying out the Friedel-Crafts acylation in aromatic solvents at a temperature from -20 to +80°C.

2. The process as claimed in claim 1, wherein the aromatic solvent is one which under the process conditions is acylated significantly more slowly than 4-chlorobiphenyl.

3. The process as claimed in claim 1 or 2, wherein the aromatic solvent is a bromoaromatic, a chloroaromatic, benzonitrile or nitrobenzene.

4. The process as claimed in at least one of claims 1 to 3, wherein the aromatic solvent is o-dichlorobenzene, chlorobenzene or p-chlorotoluene.

5. The process as claimed in at least one of claims 1 to 4, wherein AlCl₃ is employed as the Friedel-Crafts catalyst.

6. The process as claimed in at least one of claims 1 to 5, wherein, after the Friedel-Crafts acylation, hydrolysis with water, aqueous hydrochloric acid or aqueous sulfuric acid is carried out.

7. The process as claimed in at least one of claims 1 to 6, wherein 4-chlorobiphenyl and itaconic anhydride are dissolved in the aromatic solvent and this solution is added to a suspension of the Friedel-Crafts catalyst in the aromatic solvent.

8. The process as claimed in at least one of claims 1 to 6, wherein 4-chlorobiphenyl and itaconic anhydride are added in solid form to a suspension of the Friedel-Crafts catalyst in the aromatic solvent

9. The process as claimed in claim 7 or 8, wherein the addition time is between 1 minute and 12 hours, preferably between 0.5 and 4 hours.

10. The process as claimed in claim 9, wherein after completion of the addition stirring is carried out for 0.5 to 24 hours, preferably 1 to 6 hours, at a temperature between 0 and 55°C.

11. The process as claimed in at least one of claims 1 to 10, wherein the product present suspended in the solvent after the hydrolysis is filtered off.

12. The process as claimed in claim 11, wherein the mother liquor remaining after the filtration is washed with a solution of a multidentate complexing agent.

13. The process as claimed in claim 12, wherein the mother liquor washed with a solution of a multidentate complexing agent is concentrated and the product present suspended in the solvent is filtered off.

## Revendications

1. Procédé de préparation d'acide 4-(4'-chlorobiphényl-4-yl)-4-céto-2-méthylènebutyrique de formule (I) 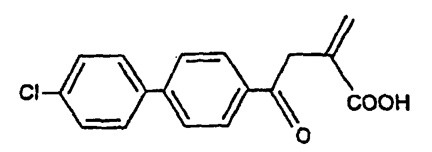 par acylation de Friedel-Crafts de 4-chlorobiphényle avec de l'anhydride d'acide itaconique, **caractérisé en ce que** l'acylation de Friedel-Crafts s'effectue dans des solvants aromatiques à une température de -20 à +80°C.

2. Procédé selon la revendication 1, **caractérisé en ce que** le solvant aromatique est un solvant qui s'acyle de façon essentiellement plus lente que le 4-chlorobiphényle dans les conditions du procédé.

3. Procédé selon la revendication 1 ou 2, **caractérisé en ce que** le solvant aromatique est un composé aromatique bromé, un composé aromatique chloré, le benzonitrile ou le nitrobenzène.

4. Procédé selon au moins l'une des revendications 1 à 3, **caractérisé en ce que** le solvant aromatique est l'o-dichlorobenzène, le chlorobenzène ou le p-chlorotoluène.

5. Procédé selon au moins l'une des revendications 1 à 4, **caractérisé en ce que** l'on utilise du AlCl₃ comme catalyseur de Friedel-Crafts.

6. Procédé selon au moins l'une des revendications 1 à 5, **caractérisé en ce que**, après l'acylation de Friedel-Crafts, on effectue une hydrolyse avec de l'eau, de l'acide chlorhydrique aqueux ou de l'acide sulfurique aqueux.

7. Procédé selon au moins l'une des revendications 1 à 6, **caractérisé en ce que** l'on dissout du 4-chlorobiphényle et de l'anhydride d'acide itaconique dans un solvant aromatique et on effectue une addition dosée de cette solution à une suspension du catalyseur de Friedel-Crafts dans le solvant aromatique.

8. Procédé selon l'une au moins des revendications 1 à 6, **caractérisé en ce que** l'on effectue une addition dosée de 4-chlorobiphényle et d'anhydride d'acide itaconique sous forme solide dans une suspension du catalyseur de Friedel-Crafts dans le solvant aromatique.

9. Procédé selon la revendication 7 ou 8, **caractérisé en ce que** la durée de l'addition dosée est comprise entre 1 minute et 12 heures, de préférence entre 0,5 et 4 heures.

10. Procédé selon la revendication 9, **caractérisé en ce que**, après la fin de l'addition dosée, on continue à agiter pendant 0,5 à 24 heures, de préférence pendant 1 à 6 heures, à une température comprise entre 0 et 55°C.

11. Procédé selon au moins l'une des revendications 1 à 10, **caractérisé en ce que**, après l'hydrolyse, on sépare par filtration le produit se trouvant en suspension dans le solvant.

12. Procédé selon la revendication 11, **caractérisé en ce que**, après la filtration, on lave la liqueur mère restante avec une solution d'un agent complexant polycoordiné.

13. Procédé selon la revendication 12, **caractérisé en ce que** l'on concentre la liqueur mère lavée avec une solution d'un agent complexant polycoordiné, et on sépare par filtration le produit se trouvant en suspension dans le solvant.
